# EUROPEAN PATENT APPLICATION

(11) **EP 3 910 064 A1**
(43) Date of publication of application: **17.11.2021**
(21) Application number: 21169201.7
(22) Date of filing: 19.04.2021
(51) Int. Cl.: C12P 7/22, A61K 36/03, A61K 8/9711, A61Q 19/08, A61K 31/335

(54) **PROCESS OF OBTAINING A PHLOROTANNIN-ENRICHED EXTRACT HAVING ANTI-ENZYMATIC ACTION FOR USE IN DERMATOLOGY**

(30) Priority: 14.05.2020 PT 2020116385
(71) Applicant: Instituto Politécnico De Leiria, 2411-901 Leiria (PT)
(72) Inventor: MARTINS DA SILVA, JOANA RITA, 2240-333 FERREIRA DO ZÊZERE (PT); DA MAIA ALVES, CELSO MIGUEL, 2240-334 FERREIRA DO ZÊZERE (PT); GONÇALVES PINTEUS, SUSETE FILIPA, 2540-422 ROSSIO DO CARVALHAL (PT); MENDES MARTINS, ALICE ISABEL, 2770-120 PAÇO DE ARCOS (PT); FREIRE FREITAS, RAFAELA PATRÍCIA, 3100-325 POMBAL (PT); PINTO PEDROSA, RUI FILIPE, 2500-930 NADADOURO (PT)
(74) Representative: Monteiro Alves, Inês

(57) **Abstract**

The present invention falls under the sector of biotechnology and concerns a process of obtaining a phlorotannin-enriched extract obtained from seaweed, preferably *Fucus spiralis.* Another object of the present invention is the phlorotannin-enriched extract obtained from the referred process, having anti-enzymatic action for application in dermatological formulations that slow down skin aging, specifically by inhibiting the activity of the enzymes collagenase and elastase. The extract obtained from the fractionation of *Fucus spiralis,* object of the present invention, is an ethyl acetate extract whose inhibitory effect (IC₅₀) on the elastase is 3.0 *µ*g/mL for a confidence interval between 2.54-3.57 *µ*g/mL and an inhibitory effect (IC₅₀) on the collagenase of 0.037 *µ*g/mL for a confidence interval between 0.009-0.142 *µ*g/mL.

## Description

### TECHNICAL FIELD

The present invention falls under the sector of biotechnology and concerns the process of obtaining a phlorotannin-enriched extract obtained from seaweed, preferably *Fucus spiralis.* Another object of the present invention is the phlorotannin-enriched extract obtained by the process, having anti-enzymatic action for application in dermatological formulations that slow down skin aging, specifically by inhibiting the activity of the enzymes collagenase and elastase.

Metalloproteinases (MMPs) are enzymes responsible for the degradation of proteins present in the cell matrix. At the dermal level, collagenase destroys collagen fibers and elastase destroys elastin, so that the natural replacement of these fibers can occur in the human body. However, over time, extra and intracellular stimuli may occur that lead to a disorganized metabolism, destroying in an exacerbated way the structural basis of the dermis, thus contributing to skin aging.

Hence, the importance of research into new bioactive ingredients that can inhibit the effect of these enzymes, the main objective of the present invention.

As age progresses, the human body stops producing elastin, and collagen begins to lose its elasticity and weaken, giving rise to one of the main external signs of aging, wrinkles. To combat skin aging, a healthy diet including foods that can slow the degradation of elastin and collagen is then essential. Currently, there are numerous studies that indicate that a diet rich in antioxidants can contribute significantly to slow down the process of cellular aging of the skin, due to their ability to inhibit the occurrence of enzymatic reactions that are the cause of reduced elastin and collagen, namely through the action of enzymes elastase and collagenase.

Moreover, continuous exposure to ultra-violet light promotes increased levels of reactive oxygen species in cells, contributing, among other factors, to increased production of elastase and collagenase, as well as to the occurrence of inflammatory processes that promote thinner skin, damage and sagging, resulting in wrinkles and other signs of aging. For the reasons presented, these enzymes have been the subject of preferential studies for the development of dermatological products, in particular topical formulations.

The constitution of seaweeds comprises several functional components, such as amino acids and essential proteins, carbohydrates, pigments, minerals, polysaccharides, dietary fibers, vitamins, polyunsaturated fatty acids, phenolic compounds and other bioactive secondary metabolites, with great potential to be used in innovative products. Among seaweeds, several disclosures have demonstrated the high nutritional and functional value of the *Fucus* genus, described as an excellent source of fibers and minerals in the diet, with a high variety of bioactive compounds such as fucoidans, phlorotannins, fucoxanthin, among others, with numerous biological activities, including antioxidant, anti-inflammatory, anti-tumoral, antimicrobial and others.

The phlorotannin-enriched extract, object of the present invention, is preferably extracted from the alga *Fucus spiralis.* This is a brown seaweed which is found in rocky substrates in the intertidal zone in protected or moderately exposed margins. It can grow up to a maximum size of 40 cm and live up to four years. This species is broadly distributed in the North Atlantic (Iceland, Norway, Denmark, the Netherlands, United Kingdom, Ireland, Atlantic coast of France, Spain, Morocco, Azores, east coast of America, from New Jersey to Nova Scotia) [1]. In Azores, it is essentially commercialized for dietary purposes clearly supporting the commercialization potential of this alga for new applications such as the one presented in this proposal.

It should also be noted that the fact that the *Fucus spiralis* seaweed, preferably selected for obtaining the extract, is also edible, represents a toxicological advantage and is expected to be used safely for dermatological purposes. In Azores, this species is considered an appetizer and known as "sea lupin", due to the terminal reproductive portions of its shoots being collected and consumed fresh [2].

Thus, in addition to the known advantageous antioxidant, anti-inflammatory, anti-tumoral, and antimicrobial properties of the species *Fucus spiralis,* the main objective of the present invention is the development of solutions providing new bioactive ingredients that can inhibit the effect of these enzymes and with dermatological usage.

### BACKGROUND ART

The application of extracts of macroalgae with inhibitory effects on the enzymes hyaluronidase, collagenase and elastase, responsible for the degradation of the skin matrix, has already been disclosed for various species of brown algae.

The present invention has close prior arts in the application of other species of brown algae, specifically the species of *Fucus, Fucus vesiculosus,* which has been exploited for many years as a source of ingredients for dermatological application. It is verified that the use of this species, although having positive results, presents the disadvantage of already being widely exploited at an industrial level, so the species selected in the present invention, besides being widely available in nature, also has a high antioxidant power, strongly contributing to its application in dermocosmetics, due to its antioxidant, anti-enzymatic, photoprotective properties and maintenance of the skin's natural microbiome.

Moreover, the studies disclosed were performed with the species *Fucus vesiculosus* essentially focusing on the anti-enzymatic activity of sulfated polysaccharides, specifically fucoidans, so they differ from the present invention in that they do not concern extracts enriched with phlorotannins.

### SUMMARY OF THE INVENTION

The objective of the present invention is to obtain an extract with anti-enzymatic action with potential for dermatological application, more specifically an extract obtained from an alga with an anti-enzymatic action on the enzymes collagenase and elastase, responsible for the degradation of the skin matrix associated to skin aging and formation of wrinkles.

The present invention therefore refers to a process of obtaining a phlorotannin-enriched extract beginning with the harvest of algae of the *Fucus* genus, preferably of the species *Fucus spiralis,* followed by an extraction of the dried and pulverized biomass in *Soxhlet,* using ethanol/water at 70%, followed by evaporation to dryness of the hydro-alcoholic solution obtained, resuspension of the dry extract resulting from the evaporation and, lastly, the fractionation of the components into different extracts, with the obtention of a phlorotannin-enriched ethyl acetate extract.

The present invention also refers to an ethyl acetate extract of *Fucus,* phlorotannin-enriched, preferably an extract of *Fucus spiralis.*

In a preferred embodiment of the invention, the ethyl acetate extract of *Fucus spiralis* phlorotannin-enriched contains Trifucol, Tetrafucol or Fucodiphloroethol or isomers thereof, Trifucophlorethol or isomers thereof, Hexafucol or isomers thereof, Difucotetraphloroethol or Trifucotriphloethol or isomers thereof and Tetrafucotetraphlorethol or Pentafucodiphlorethol or Hexafucophlorethol or isomers thereof.

In a more preferred embodiment of the invention the phlorotannin-enriched extract of *Fucus spiralis* presents an IC₅₀ (concentration of the drug that induces half of the maximum effect) of 0.037 *µ*g/mL on the collagenase for a confidence interval of 0.009-0.142 *µ*g/mL and an IC₅₀ of 3.0 *µ*g/mL on the elastase for a confidence interval of 2.54-3.57 *µ*g/mL. The inhibitory effect of the extract on the collagenase and elastase is superior to that noted in other species of algae used and reported in literature.

### BRIEF DESCRIPTION OF DRAWINGS

Figure 1 - Cytotoxic activity of the ethyl acetate extract of *Fucus spiralis,* phlorotannin-enriched, in keratinocytes (HaCat) in a concentration of 1 mg/mL.
Figure 2 - Inflammatory activity of the ethyl acetate extract of *Fucus spiralis,* phlorotannin-enriched, in a concentration of 300 *µ*g/mL in a cellular model of the type RAW264.7 and using lipopolysaccharides as positive control.

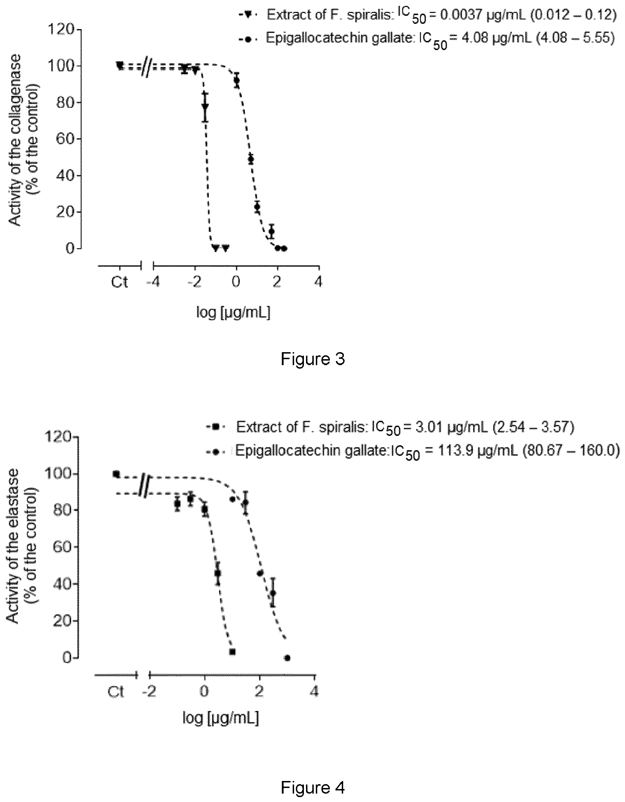

Figure 3 - Anti-collagenase activity of the ethyl acetate extract of *Fucus spiralis,* phlorotannin-enriched, in a confidence interval of 0.009-0.142 *µ*g/mL and using epigallocatechin gallate as reference compound.
Figure 4 - Anti-elastase activity of the ethyl acetate extract of *Fucus spiralis,* phlorotannin-enriched, in a confidence interval of 2.54-3.57 *µ*g/mL and using epigallocatechin gallate as reference compound.

### DESCRIPTION OF EMBODIMENTS

Below is a description of the various execution steps of the invention as well as a demonstration of the results corroborating the anti-enzymatic action of the phlorotannin-enriched extract.

### Preparing the algal biomass

To obtain a composition containing phlorotannins, the macroalgae *Fucus spiralis* is harvested. For information purposes, the assays to be demonstrated in the next sections correspond to the *Fucus spiralis* macroalgae harvested on the Portuguese coast, more specifically on Consolação beach, in Peniche, on October 1, 2019. Then, the fresh seaweed is washed, first with sea water and then with distilled water, in order to remove invertebrate organisms, sand and other marine contaminants; it is then weighed, packed, frozen at - 80°C and freeze-dried. Finally, the seaweed, now dry algal biomass, is pulverized and stored in a cool, dry and dark place, where it remains until the extraction stage.

### Extraction

For the extraction of phlorotannins with a high degree of purity, that is, without the presence, or with a reduced percentage of other groups of compounds existing in macroalgae, a dry and pulverized biomass extraction of |*Fucus spiralis* was carried out in *Soxhlet,* using ethanol/water (70%) as solvent. After exhaustion, the hydro-alcoholic solution obtained was evaporated to dryness, under vacuum, at 40°C, in a rotary evaporator.

The extraction method described was selected due to the fact of being an extraction method that allows a prior sorting of different groups of compounds present in the algae. Upon proceeding with the fractionation, each of the extracts is enriched in different groups of constituents, according to their affinity to each of the solvents used.

### Extracted fractions

Subsequently, the resulting dry extract was resuspended in Milli-Q water at about 80°C and homogenized. After cooling to room temperature, the suspension was filtered in filter paper (n°4), having obtained two fractions: solid (retained in the filter paper) and aqueous (collected in an Erlenmeyer balloon). The aqueous fraction was submitted to a sequential liquid/liquid (L/L) extraction, first with ethyl ether and then with ethyl acetate. The organic phases were dried with sodium sulfate, filtered, and evaporated to dryness in a rotary evaporator. The remaining aqueous fraction was frozen and freeze-dried. This sequence of extractions allowed the fractionation of the different components of *Fucus spiralis,* resulting in four extracts of distinct chemical composition:
- apolar solid extract;
- ethyl ether extract;
- ethyl acetate extract; and
- polar aqueous extract.

### Characterization of the ethyl acetate extract of Fucus spiralis

A qualitative analysis of the phlorotannin-enriched extract was carried out by high-performance liquid chromatography coupled to mass spectrometry (LCMS). The spectral data obtained were compared with those from literature.

According to literature, many studies resort to the use of tandem mass spectrometry (LCMS/MSⁿ) to identify this group of compounds. In the present case, LCMS was used, the mass spectra having been acquired in the positive [M+H]⁺ and negative [M-H]⁻ ion modes in order to confirm the molecular mass corresponding to each peak.

**Table 1 - Attempt to identify phlorotannins present in the ethyl acetate extract of Fucus spiralis.**

| **M** (*m*/*z*) | **[M-H]⁻** (*m*/*z*) | **[M+H]⁺** (*m*/*z*) | **Other ions** | **Identification attempt** | **References** |
|---|---|---|---|---|---|
| 374 | 373 | 375 | - | Trifucol | [3-5] |
| 498 | 497 | 499 | - | Tetrafucol or Fucodiphloroethol or isomers thereof | [4-7] |
| 498 | 497 | 499 | | | |
| 622 | 621 | 623 | - | Trifucophlorethol or isomers thereof | [3-5] |
| 746 | 745 | 747 | 1493 [2M+H]⁺ * | Hexafucol or isomers thereof | [5-8] |
| 746 | 745 | 747 | 1493 [2M+H]⁺ * | | |
| 870 | 869 | 871 | | Difucotetraphloroethol or Trifucotriphloethol or isomers thereof | [4-7] |
| 870 | 869 | 871 | | | |
| 994 | 993 | 995 | 496 [M-2H]2- | Tetrafucotetraphlorethol | [5-7] |
| 994 | 993 | 995 | 496 [M-2H]2- | or Pentafucodiphlorethol or Hexafucophlorethol or isomers thereof | |
| 994 | 993 | 995 | - | | |

The methodology used for obtaining the phlorotannin-rich extract allowed this group of compounds to be obtained with a high degree of purity and good chemical stability, since no changes in chemical profile were observed over time, when evaluated by thin layer chromatography and high-performance liquid chromatography.

### Evaluation of the biological potential of the extracts

The biological potential was analyzed for each extract, more specifically the inhibition of collagenase, the inhibition of elastase, the antioxidant potential (inhibition of reactive oxygen species production), non-cytotoxic potential, photoprotective, and non-inducing inflammation.

The inhibitory effect of the different obtained extracts (IC₅₀ - concentration of the drug that induces half of the maximum effect) was evaluated according to a certain range of concentrations tested, on the enzymes collagenase and elastase, for each obtained extract, using specific analytical kits for each of the enzymes, and following the manufacturer's specifications, demonstrated in the following table results.

**Table 2 - Inhibitory effect (IC₅₀) of the different extracts obtained based on the interval of concentrations tested on the enzymes collagenase and elastase.**

| Extract | IC₅₀ Collagenase [*µ*g/mL] | IC₅₀ Elastase [*µ*g/mL] |
|---|---|---|
| Ethanolic | 391.6 (328.70-466.60) | >1000 |
| Ethyl acetate | 0.037 (0.009-0.142) | 3.01 (2.54-3.57) |
| Aqueous | 31.3 (28.40-34.50) | 586.5 (445.80 - 771.60) |
| Epigallocatechin gallate (reference compound) | 4.8 (4.10-5.50) | 113.9 (80.70-160.00) |

The ethyl ether extract was not tested due to the low yield obtained from the extraction.

The results of the anti-enzymatic activity were analyzed bearing in mind the IC₅₀ values obtained.

It was noted that the ethyl acetate extract, in very low concentration, is a potent inhibitor of collagenase with an IC₅₀ of 0.037 *µ*g/mL for a confidence interval of 0.009-0.142 *µ*g/mL and of elastase with an IC₅₀ = 3.0 *µ*g/mL for a confidence interval of 2.54-3.57 *µ*g/mL.

The results obtained are highly relevant when compared to the inhibitory activity of an extract of *Fucus vesiculosus* (containing 60% of fucoidan and 30% of polyphloroglucinol), on the enzymes elastase (IC₅₀ = 76 *µ*g/mL) and collagenase (IC₅₀ = 60 *µ*g/mL) reported by [9]

**Table 3 - Comparison between the inhibitory effect of the ethyl acetate extract of Fucus spiralis and an extract of Fucus vesiculosus on the enzymes collagenase and elastase.**

| Extract | IC₅₀ Collagenase [*µ*g/mL] | IC₅₀ Elastase [*µ*g/mL] |
|---|---|---|
| Ethyl acetate extract - *Fucus spiralis* | 0.037 (0.009-0.142) | 3.0 (2.5-3.6) |
| *Fucus vesiculosus* | 76.0 | 60.0 |

### Evaluation of the cytotoxic activity of the ethyl acetate extract

The cytotoxic activity of the ethyl acetate extract was evaluated in an *in vitro* model of human keratinocytes (cells HaCaT), as described ahead.

The HaCaT cells were maintained in RPMI 1640 medium supplemented with 10% bovine serum, 100 U of penicillin G, 100 *µ*g of streptomycin, 2mM L-glutamine and maintained at 95% humidity and 5% CO₂ at 37°C. The cells were grown on 96 - wells plates at a density of 4x10⁴ cells/well) and incubated overnight. After this period, the cells were treated with the extract at a concentration of 1000 *µ*g/mL for 24 hours. The cytotoxic effect was estimated by the MTT (3-[4,5-dimethylthiazol-2-yl]-2,5-diphenyltetrazolium bromide) colorimetric assay.

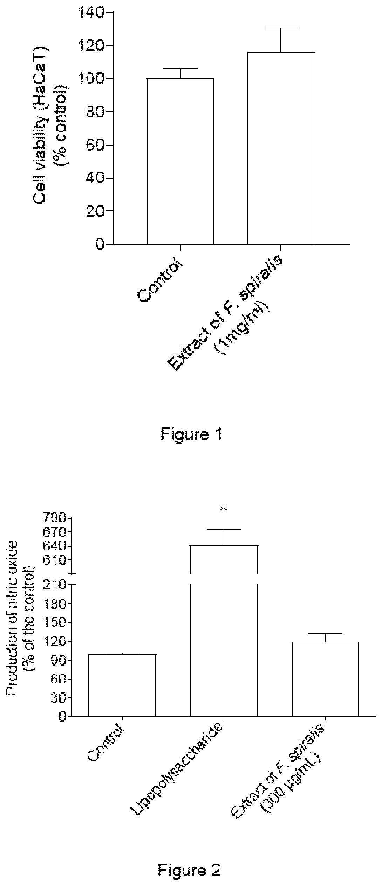

The fact that the extract, at the concentration mentioned, does not reveal cytotoxicity in an *in vitro* model of human keratinocytes, and as shown by the graph in Figure 1, is a first indicator that there will be no toxicity to the skin, more specifically in the epidermis.

The inflammatory activity of the extract was evaluated in the cell model RAW264.7 in order to verify whether it stimulated an inflammatory response through the quantification of nitric oxide. The cells RAW264.7 were grown on 96 - wells plates at a density of 5x10⁴ cells/well and incubated overnight. After this period, the cells were treated with phlorotannin-enriched extract at a non-toxic concentration (300 *µ*g/mL) to assess whether the extract induced an inflammatory response. This response was evaluated by quantifying nitric oxide levels. Lipopolysaccharide was used as a positive control. It should also be noted that the extract did not reveal cytotoxicity at concentrations below 300 *µ*g/mL in the tested cellular models, nor did stimulate the production of nitric oxide. The results are shown in the graph of Figure 2.

If the extract will induce inflammation in the studied model, it could not be used in dermatological formulations. The results obtained in the *in vitro* assays allowed to conclude that, below 300 *µ*g/mL, the extract did not induce an inflammatory process in the studied cells. This is additional information that supports the safety of the extract for dermatological application.

In addition to the marked anti-enzymatic activity shown by this phlorotannin-enriched extract, it was also possible to observe a high antioxidant power, as well as a powerful ability to reduce the production of reactive oxygen species (ROS) in the cellular model of keratinocytes (HaCaT) when exposed to hydrogen peroxide or ultraviolet radiation.

All these effects were observed at non-cytotoxic concentrations. These results suggest that the use of this extract in dermatological formulations may present multiple biological activities that contribute to slowing down skin aging.

Therefore, the phlorotannin-enriched extract, object of the present invention, is used in the inhibition of collagenase and elastase activity and used in the prevention of skin aging and in the treatment of wrinkles.

A dermatological formulation containing the phlorotannin-enriched extract is used as sole active ingredient or in combination with one or more active ingredients and used in the prevention of skin aging and treatment of wrinkles.

### REFERENCES

[1] White, N. 2008. Fucus spiralis Spiral wrack. In Tyler- Walters H. and Hiscock K. (eds) Marine Life Information Network: Biology and Sensitivity Key Information Reviews, [on-line]. Plymouth: Marine Biological Association of the United Kingdom. [cited 12-04-2020]. Available from: https://www.marlin.ac.uk/species/detail/1337
[2] Neto AI, Tittley I, Raposeiro PM (2005) Flora Marinha do Litoral dos Açores. Secretaria Regional do Ambiente and do Mar, 148 pp.
[3] Lopes, G.; Barbosa, M.; Vallejo, F.; Gil-Izquierdo, Á.; Andrade, P.B.; Valentão, P.; Pereira, D.M.; Ferreres, F. Profiling phlorotannins from Fucus spp. of the Northern Portuguese coastline: Chemical approach by HPLC-DAD-ESI/MSn and UPLC-ESI-QTOF/MS. Algal Research 2018, 29, 113-120.
[4] Hermund, D.B.; Plaza, M.; Turner, C.; Jonsdottir, R.; Kristinsson, H.G.; Jacobsen, C.; Nielsen, K.F. Structure dependent antioxidant capacity of phlorotannins from Icelandic Fucus vesiculosus by UHPLC-DAD-ECD-QTOFMS. Food Chemistry 2018, 240, 904-909.
[5] Catarino, M.D.; Silva, A.M.S.; Mateus, N.; Cardoso, S.M. Optimization of Phlorotannins Extraction from Fucus vesiculosus and Evaluation of Their Potential to Prevent Metabolic Disorders. Marine Drugs 2019, 17, 162.
[6] Ferreres, F.; Lopes, G.; Gil-Izquierdo, A.; Andrade, P.B.; Sousa, C.; Mouga, T.; Valentão, P. Phlorotannin Extracts from Fucales Characterized by HPLC-DAD-ESI-MSn: Approaches to Hyaluronidase Inhibitory Capacity and Antioxidant Properties. Marine Drugs 2012, 10, 2766-2781.
[7] Tierney, M.S.; Soler-Vila, A.; Rai, D.K.; Croft, A.K.; Brunton, N.P.; Smyth, T.J. UPLC-MS profiling of low molecular weight phlorotannin polymers in Ascophyllum nodosum, Pelvetia canaliculata and Fucus spiralis. Metabolomics 2014, 10, 524-535.
[8] Pan, H. A non-covalent dimer formed in electrospray ionisation mass spectrometry behaving as a precursor for fragmentations. Rapid Communications in Mass Spectrometry 2008, 22, 3555-3560.
[9] Fitton, J.H., Dell'Acqua, G., Gardiner, V-A., Karpiniec, S.S.,Stringer, D.N., Emma Davis, E.Topical Benefits of Two Fucoidan-Rich Extracts from Marine Macroalgae.Cosmetics 2015, 2, 66-81; doi:10.3390/cosmetics2020066

## Claims

1. A process of obtaining a phlorotannin-enriched extract **characterized by** comprising the following steps:
a) harvesting algae of the *Fucus* genus;
b) extracting a dry and pulverized biomass of the alga harvested from *Fucus* in *Soxhlet,* using ethanol/water at 70%;
c) evaporating to dryness, under vacuum, of a hydro-alcoholic solution obtained in step b) at a temperature of 40°C;
d) resuspending a dry extract obtained in step c) in Milli-Q water at 80 °C;
e) fractionating a suspension containing the components of *Fucus* in an apolar solid extract, an ethyl ether extract, an ethyl acetate extract and a polar aqueous extract;
f) obtaining a phlorotannin-enriched ethyl acetate extract.

2. The process of obtaining a phlorotannin-enriched extract according to claim 1 **characterized in that** the alga used in step a) is *Fucus spiralis.*

3. The process of obtaining a phlorotannin-enriched extract according to preceding claim **characterized in that** the extract obtained in step f) contains Trifucol, Tetrafucol or Fucodiphloroethol or isomers thereof, Trifucophlorethol or isomers thereof, Hexafucol or isomers thereof, Difucotetraphloroethol or Trifucotriphloethol or isomers thereof and Tetrafucotetraphlorethol or Pentafucodiphlorethol or Hexafucophlorethol or isomers thereof.

4. A phlorotannin-enriched extract obtained by the process described in claims 1 to 3 **characterized by** being an ethyl acetate extract of algae of the *Fucus* genus.

5. The phlorotannin-enriched extract according to the preceding claim **characterized by** being an extract of *Fucus spiralis.*

6. The phlorotannin-enriched extract according to the preceding claim **characterized by** containing Trifucol, Tetrafucol or Fucodiphloroethol or isomers thereof, Trifucophlorethol or isomers thereof, Hexafucol or isomers thereof, Difucotetraphloroethol or Trifucotriphloethol or isomers thereof and Tetrafucotetraphlorethol or Pentafucodiphlorethol or Hexafucophlorethol or isomers thereof.

7. The phlorotannin-enriched extract according to claim 5 **characterized by** presenting an IC₅₀ of 0.037 *µ*g/mL on the collagenase for a confidence interval of 0.009-0.142 *µ*g/mL and an IC₅₀ of 3.0 *µ*g/mL on the elastase for a confidence interval of 2.54-3.57 *µ*g/mL.

8. The phlorotannin-enriched extract according to claims 4 to 7 for use in inhibiting collagenase and elastase activity.

9. The phlorotannin-enriched extract according to claims 4 to 7 for use in the prevention of skin aging and in the treatment of wrinkles.

10. A dermatological formulation containing the phlorotannin-enriched extract according to claims 4 to 7 as a sole active ingredient or in combination with one or more active ingredients.

11. A phlorotannin-enriched extract according to claims 4 to 7 for use as a cometic in the prevention of skin aging and treatment of wrinkles.

12. A dermatological formulation according to claim 10 for use in the prevention of skin aging and treatment of wrinkles.
